# EUROPÄISCHE PATENTANMELDUNG

(11) **EP 2 772 150 A1**
(43) Veröffentlichungstag der Anmeldung: **03.09.2014**
(21) Anmeldenummer: 14000649.5
(22) Anmeldetag: 24.02.2014
(51) Int. Cl.: A41C 3/00, A61F 13/14

(54) **Kompressions-BH nach Ablatio**

(30) Priorität: 28.02.2013 DE 202013001952 U
(71) Anmelder: Edelweiss Basics GmbH & Co. Kg, 83098 Brannenburg (DE)
(72) Erfinder: Weber-Unger, Georg, 6330 Kufstein (AT)
(74) Vertreter: Kador & Partner

(57) **Zusammenfassung**

Kompressionsbüstenhalter (1), dadurch gekennzeichnet, dass der Kompressionsbüstenhalter (1) ein im Tragezustand um den Thorax der Trägerin angeordnetes durchgehendes Kompressionsband (3) umfasst, wobei das Kompressionsband (3) eine Aussparung (4), die einen Freiraum für eine Brust bildet, aufweist.

## Beschreibung

Die Erfindung bezieht sich auf einen Kompressionsbüstenhalter.

Kompressionsbüstenhalter werden zur Nachsorge im Anschluss an eine operative Entfernung der Brust eingesetzt. Die Kompressionsbüstenhalter werden post-operativ getragen um Druck beziehungsweise Kompression auf die entstandene Wunde und das angrenzende Brustgewebe auszuüben und somit zur Heilung beizutragen. Insbesondere wird dadurch einem Lymphstau im Bereich des operierten Gewebes entgegengewirkt.

Aus der DE 20 2008 006 847 U1 ist ein Kompressionsbüstenhalter bekannt, der an unterschiedlich große Brüste anpassbar ist. Plastische Operationen, insbesondere Brustrekonstruktionen im Anschluss an eine Ablatio, führen in der Folge häufig zu Unterschieden in Form und Größe von operierter und gesunder Brust. Die Anpassung des Kompressionsbüstenhalters wird dadurch erreicht, dass der Büstenhalter aus zwei lösbar miteinander verbundenen Seitenteilen besteht. Hierbei kann jedes Seitenteil an die Größe der zugeordneten Brust angepasst werden.

Ein wesentlicher Nachteil der bekannten Kompressionsbüstenhalter besteht darin, dass sich beim Tragen eine ungleichmäßige Kompression auf die durch operative Entfernung der einen Brust entstandene Wunde einstellt. Durch die Ausgestaltung der bekannten Kompressionsbüstenhalter liegt der Büstenhalter beim Tragen nicht oder nicht vollständig an der operierten Seite an, sondern es bildet sich im Bereich der entstandenen Wunde und insbesondere im Zwischenbrustbereich ein Hohlraum. Der erforderliche Mindestdruck auf die Wunde und das umgebende Gewebe zur Vermeidung eines Lymphstaus ist dadurch nicht gegeben. Gleichzeitig drückt der Büstenhalter auf die vorhandene gesunde Brust. Es entsteht dadurch eine ungleichmäßige Kompression auf die Wunde der operierten Seite. Zugleich wird die vorhandene, nicht-operierte Brust verstärkt komprimiert. Diese ungleichmäßige Kompression wirkt sich negativ auf die post-operative Versorgung aus und verursacht darüber hinaus ein unbequemes Tragegefühl.

Ein weiterer Nachteil besteht darin, dass die bekannten Kompressionsbüstenhalter verrutschen können und damit die erforderliche Positionierung nicht gewährleistet ist.

Der Erfindung liegt die Aufgabe zugrunde, einen Kompressionsbüstenhalter bereitzustellen, der die oben erwähnten Nachteile vermeidet, und welcher beim Tragen eine gleichmäßige Kompression auf die Wunde sowie ein bequemes Tragegefühl gewährleistet.

Dies wird erfindungsgemäß mit einem Kompressionsbüstenhalter gelöst, der dadurch gekennzeichnet ist, dass der Kompressionsbüstenhalter ein im Tragezustand um den Thorax der Trägerin angeordnetes durchgehendes Kompressionsband umfasst, wobei das Kompressionsband eine Aussparung, die einen Freiraum für eine Brust bildet, aufweist.

Der vorliegenden Erfindung liegt die Erkenntnis zugrunde, dass die Aufgabe durch ein durchgehendes Kompressionsband gelöst wird.

Durchgehend im Sinne dieser Anmeldung meint, dass das Kompressionsband rundum verläuft, an oder nahe den Verschlussteilen, falls solche vorhanden sind, beginnt, aber auch, dass das Kompressionsband teilweise umfänglich von nicht oder wenig elastischem Material ersetzt sein kann. Dies beeinträchtigt die Funktion nicht, solange die nötige Kompression erreicht wird und ansonsten der Tragekomfort gewährleistet wird.

Durch den erfindungsgemäßen Kompressionsbüstenhalter wird eine gleichmäßige Kompression beziehungsweise ein gleichmäßiger Druck auf die durch die Entfernung der Brust entstandene Wunde und des umliegenden Gewebes auszuüben. Der erfindungsgemäße Kompressionsbüstenhalter verhindert zudem, dass sich im Bereich der entstandenen Wunde und insbesondere im Zwischenbrustbereich ein Hohlraum bildet. Ferner gewährleistet der Kompressionsbüstenhalter die erforderliche Mindestkompression. Dadurch wird einem Lymphstau im Bereich des operierten Gewebes entgegengewirkt. Ein weiterer Vorteil des erfindungsgemäßen Kompressionsbüstenhalters ist, dass ein bequemes Tragegefühl ermöglicht wird und die vorhandene Brust gestützt wird, aber auf diese kein Druck ausgeübt wird. Mit dem erfindungsgemäßen Kompressionsbüstenhalter werden ein bequemes Tragegefühl und eine sichere Positionierung des Kompressionsbüstenhalters am Körper der Trägerin gewährleistet.

Das Kompressionsband des erfindungsgemäßen Kompressionsbüstenhalter ist zumindest in seiner den Thorax umgebenden Längsausdehnung zum Teil elastisch, und vorzugsweise in seiner Längsausdehnung und in seiner Querausdehnung zum Teil elastisch. Besonders bevorzugt ist das Kompressionsband in seiner den Thorax umgebenden Längsausdehnung und/oder seiner Querausdehnung komplett elastisch.

Die Aussparung des Kompressionsbüstenhalters ist in seiner Lage sowie in der Form und/oder der Größe der Brust angepasst. Die Aussparung ermöglicht, dass im Tragezustand die vorhandene Brust aus dem Kompressionsband vollständig hindurchtreten und damit vollständig aus diesem hervorstehen kann. Das Kompressionsmaterial liegt somit nicht auf der vorhandenen Brust, sondern direkt am Thorax der Trägerin auf. Der erfindungsgemäße Kompressionsbüstenhalter hat insbesondere die folgenden zwei Effekte. Einerseits wird vermieden, dass das Kompressionsband auf die vorhandene Brust drückt beziehungsweise diese komprimiert. Andererseits wird zugleich die Bildung eines Hohlraums im Bereich der operierten Seite sowie im Zwischenbrustbereich vermieden.

Vorzugsweise ist das Kompressionsband einstückig ausgebildet ist. Dies hat den Vorteil, dass das Kompressionsband nicht aus zwei oder mehreren Teilstücken zusammengefügt werden muss, wie beispielsweise durch Vernähen.

Der Kompressionsbüstenhalter weist vorzugsweise an seinen beiden Enden jeweils ein Verschlussteil auf, wobei die Verschlussteile einander zugeordnet und miteinander verbindbar sind. Dadurch kann der Kompressionsbüstenhalter geöffnet werden, was die Nachsorge vereinfacht.

Der Kompressionsbüstenhalter umfasst vorzugsweise ein im Tragezustand um den Thorax der Trägerin angeordnetes durchgehendes Unterbrustband. Die vorhandene Brust wird durch das Unterbrustband gestützt. Zugleich gewährleistet das Unterbrustband eine sichere Positionierung des Kompressionsbüstenhalters am Körper der Trägerin. Insbesondere ein kraniales Verrutschen oder Verschieben des Kompressionsbüstenhalters wird vermieden.

Das Unterbrustband kann beispielsweise aus Polyamid, Elastan oder einer Kombination daraus bestehen. Die Breite des Unterbrustbandes beträgt vorzugsweise 0,5 cm bis 5 cm, weiter bevorzugt 1,5 cm bis 4,5 cm, und besonders bevorzugt 2,5 cm bis 3,5 cm.

Hierbei ist besonders bevorzugt, dass das Unterbrustband entlang seiner Oberkante mit der Unterkante des Kompressionsbands durch eine Naht verbunden ist. Durch diese Verbindung erhält der Kompressionsbüstenhalter eine höhere mechanische Stabilität.

Vorzugsweise ist das Unterbrustband in seiner den Thorax umgebenden Längsausdehnung und/oder seiner Querausdehnung zumindest zum Teil elastisch, und besonders bevorzugt komplett elastisch. Durch diese Elastizität kann sich das Stützband an Umfangsänderungen des Thorax der Trägerin, wie beispielsweise Atembewegungen, anpassen und vermittelt ein bequemes Tragegefühl. Durch die Elastizität werden zudem Druckstellen vermieden.

Das Kompressionsband weist vorzugsweise unterhalb der Aussparung in seiner quer zur den Thorax umgebenden Längsausdehnung eine Breite von mindestens 0,5 cm, mehr bevorzugt eine Breite von mindestens 0,7 cm und besonders bevorzugt eine Breite von mindestens 0,9 cm auf.

Das Kompressionsband weist vorzugsweise unterhalb der Aussparung in seiner quer zur den Thorax umgebenden Längsausdehnung eine Breite von maximal 5 cm, mehr bevorzugt eine Breite von maximal 4 cm und besonders bevorzugt eine Breite von maximal 3 cm auf.

Vorzugsweise besteht der eine Verschlussteil aus einem Hakenband und der andere Verschlussteil aus einem Ösenband. Diese zwei einander zugeordneten Verschlussteile können auch aus einem Klettverschluss, aus Knöpfen und entsprechenden Knopflöchern, einem Reißverschluss oder Kombinationen davon bestehen. Die Verschlussteile können an der Vorderseite oder an der Rückseite des Kompressionsbüstenhalter angeordnet sein, wobei sie bevorzugt an der Vorderseite des Kompressionsbüstenhalter angebracht sind. Eine Anordnung an der Vorderseite des Kompressionsbüstenhalter hat den Vorteil die Nachsorge zu vereinfachen, beispielsweise beim Öffnen des Kompressionsbüstenhalter bei einer am Rücken liegenden Trägerin.

Die Aussparung ist vorzugsweise an seiner dem Thorax abgewandten Seite mit einer Stofflage abgedeckt. Die Stofflage ist dabei auf der dem Thorax abgewandten Seite des Kompressionsbandes angeordnet. Die Stofflage hat den Zweck, die vorhandene Brust abzudecken und zu schützen. Die Stofflage kann aus einem oder mehreren Materialien bestehen, wie beispielsweise Baumwolle, Viskose, Polyamid und Elastan.

Vorzugsweise wird durch die Aussparung im Kompressionsband und der Stofflage einen Aufnahmeraum für eine Brust gebildet.

Das Kompressionsband besteht vorzugsweise aus Polyester, Elastan oder einer Kombination davon. Das Kompressionsband kann vorzugsweise mit Micro-Schaum verstärkt sein. Dies hat den Vorteil, dass keine Druckstellen einstehen.

Die Elastizität des Unterbrustbandes quer zu seiner Längsausdehnung ist entweder gleich groß oder geringer ausgebildet als in seiner Längsausdehnung.

Vorzugsweise wird die Aussparung des Kompressionsbandes vollständig durch eine im Kompressionsband angeordnete Öffnungsnaht begrenzt. Die Naht soll verhindern, dass das Kompressionsband entlang der Öffnung ausfransen kann.

In einer bevorzugten Ausführungsform bedeckt die Stofflage wenigstens das gesamte Kompressionsband an seiner dem Thorax abgewandten Seite. Dazu kann die Stofflage beispielsweise an ihren Rändern ganz oder teilweise mit dem Kompressionsband und/oder dem oberen Band vernäht sein. Dadurch bildet sich zwischen der dem Thorax abgewandten Seite des Kompressionsbandes und der Stofflage ein Zwischenraum. In dieser Ausführungsform kann das Kompressionsband zusätzlich eine Prothesen-Aussparung aufweisen. Diese Prothesen-Aussparung befindet sich in jenem Vorderbereich des Kompressionsbandes, welcher auf der operierten Seite der Trägerin aufliegt. Ist also beispielsweise die Trägerin an der linken Brust operiert worden, so befindet sich die Prothesen-Aussparung im linken Vorderbereich des Kompressionsbandes, während sich die Aussparung, die einen Freiraum für die vorhandene gesunde Brust bildet, im rechten Vorderbereich des Kompressionsband befindet. Die Prothesen-Aussparung im Kompressionsband ermöglicht es, eine Brustprothese in den zwischen der dem Thorax abgewandten Seite des Kompressionsbandes und der Stofflage gebildeten Zwischenraum einzuführen.

Der Kompressionsbüstenhalter umfasst vorzugsweise zwei Träger, wobei jeweils ein Träger über jeweils eine Schulter geführt wird. Für einen erhöhten Tragekomfort können die Träger gepolstert sein. Vorzugsweise sind die Träger in ihrer Länge verstellbar. Damit wird eine genaue Anpassung der Lage und Position des Kompressionsbüstenhalters an die Trägerin ermöglicht.

Nachstehend ist die Erfindung anhand der beigefügten Figur beispielhaft näher erläutert. Darin zeigt

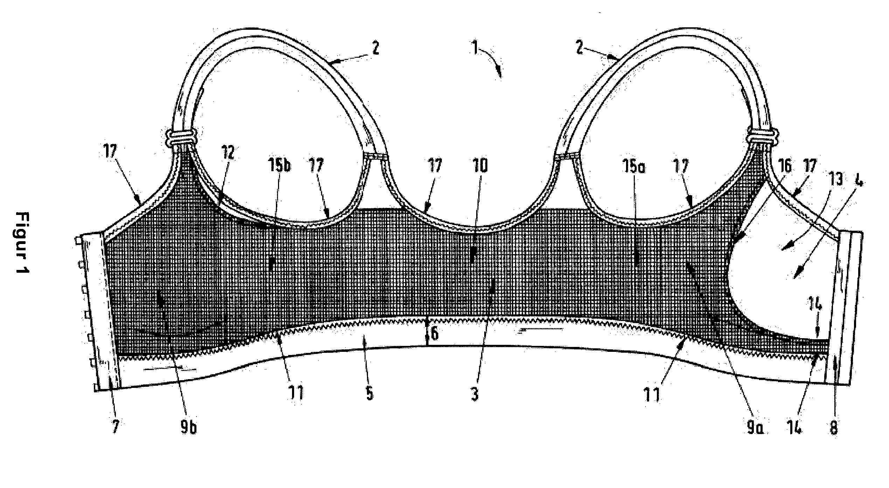

Figur 1 eine Innenansicht eines an der Vorderseite angeordnete Verschlüsse aufweisende, geöffneten Kompressionsbüstenhalters gemäß einer Ausführungsform der Erfindung.

Die in Figur 1 gezeigte bevorzugte Ausführungsform des Kompressionsbüstenhalters (1) ist für eine Trägerin bestimmt, deren rechte Brust teilweise oder vollständig operativ entfernt wurde.

Der Kompressionsbüstenhalter (1) weist zwei Vorderbereiche, nämlich einen linken Vorderbereich (9a) und einen rechten Vorderbereich (9b), sowie zwei Seitenbereiche, nämlich einen linken Seitenbereich (15a) und einen rechten Seitenbereich (15b), sowie einen Rückenbereich (10) auf.

Der Kompressionsbüstenhalter (1) umfasst ein Kompressionsband (3), welches sich vom einen Verschlussteil (7) zum anderen Verschlussteil (8) durchgehend erstreckt.

Der Kompressionsbüstenhalter (1) umfasst ferner ein oberes Band (17), welches das eine Verschlussteil (7), die Oberkante des Kompressionsbandes (3) und das andere Verschlussteil (8) verbindet. Die Oberkante des Kompressionsbandes (3) folgt dem oberen Band (17) in wenigstens einem Teil des linken Vorderbereichs (9a), des linken Seitenbereichs (15a), des Rückenbereichs (10), des rechten Seitenbereich (15b), sowie des rechten Vorderbereichs (9b) und ist mit diesen Teilen auch durch eine Naht verbunden.

Jeder Träger (2) ist mit seinen beiden Enden am oberen Band (17) befestigt.

Im gezeigten Ausführungsbeispiel der Figur 1 liegt die Aussparung (4) des Kompressionsbands (3) im linken Vorderteil (9a) des Kompressionsbüstenhalter (1). Der Kompressionsbüstenhalter (1) ist daher für eine Trägerin bestimmt, deren rechte Brust teilweise oder vollständig operativ entfernt wurde. Die Aussparung (4) wird in dieser Ausführungsform durch eine im Kompressionsband (3) angeordnete Öffnungsnaht (16) begrenzt. Die Aussparung (4) bildet einen Freiraum für die vorhandene linke Brust der Trägerin.

Der Kompressionsbüstenhalter (1) umfasst weiter einen aus zwei einander zugeordneten Verschlussteilen (7, 8) bestehenden Vorderverschluss. Wie aus Figur 1 ersichtlich, ist das durchgehende Kompressionsband (3) an seinen beiden Enden mit dem einen Verschlussteil (7) bzw. dem anderen Verschlussteil (8) verbunden. Der eine Verschlussteil (7) ist im gezeigten Beispiel ein Hakenband, während der andere Verschlussteil (8) ein Ösenband ist.

Das Kompressionsband (3) weist unterhalb der Aussparung (4) in seiner quer zur den Thorax umgebenden Längsausdehnung (14) eine Mindestbreite auf. Die Breite beträgt in diesem Beispiel 1 cm. Damit ist gewährleistet, dass das Kompressionsmaterial (3) seiner Länge nach am Thorax der Trägerin eng anliegt, die erforderliche Mindestkompression ausgeübt wird und sich eine gleichmäßige Kompression auf die Wunde und das umgebende Gewebe einstellt.

Wie aus Figur 1 weiter ersichtlich, befindet sich am unteren Rand des Kompressionsbandes (3) ein Unterbrustband (5), welches in seiner den Thorax umgebenden Länge der Länge des Kompressionsmaterials (3) entspricht. Für eine höhere mechanische Stabilität ist das Unterbrustband entlang seiner Oberkante mit der Unterkante des Kompressionsbands durch eine Naht (11) vernäht. Die Naht (11) ist in diesem Ausführungsbeispiel eine Zickzack-Naht.

In diesem Ausführungsbeispiel nach Figur 1 besteht das Kompressionsband (3) aus 94% Polyester und 6% Elastan, und das Unterbrustband (5) aus 89% Polyamid und 11 % Elastan.

Das Unterbrustband ist in seiner den Thorax umgebenden Längsausdehnung elastisch ausgebildet. Durch diese Elastizität kann sich das Unterbrustband (5) an Umfangsänderungen des Thorax der Trägerin, wie beispielsweise Atembewegungen, anpassen und vermittelt ein bequemes Tragegefühl. Zugleich gewährleistet das Unterbrustband (5) eine sichere Positionierung des Kompressionsbüstenhalters (1) am Körper der Trägerin.

Die Breite (6) des Unterbrustbandes (5) beträgt in diesem Ausführungsbeispiel 3 cm.

Wie aus Figur 1 ersichtlich, befindet sich im rechten Vorderbereich (9b) an der Oberkante des Kompressionsbandes (3) eine Prothesen-Aussparung (12). Diese Prothesen-Aussparung (12) bildet gemeinsam mit einem Teil des oberen Bandes (17) eine Öffnung. Durch diese Prothesen-Aussparung (12) kann beispielsweise eine Brustprothese in den zwischen der dem Thorax abgewandten Seite des Kompressionsbandes (3) und der Stofflage gebildeten Raum eingeführt werden.

### Bezugszeichenliste:

- **1**: Kompressionsbüstenhalter
- **2**: Träger
- **3**: Kompressionsband
- **4**: Aussparung
- **5**: Stützband
- **6**: Breite des Stützbandes
- **7**: Verschlussteil
- **8**: Verschlussteil
- **9a**: linker Vorderbereich
- **9b**: rechter Vorderbereich
- **10**: Rückenbereich
- **11**: Naht
- **12**: Prothesen-Aussparung
- **13**: Aufnahmeraum für eine Brust
- **14**: Breite des Kompressionsbandes (3) unterhalb der Öffnung
- **15a**: linkes Seitenteil
- **15b**: rechtes Seitenteil
- **16**: Öffnungsnaht
- **17**: oberes Band

## Patentansprüche

1. Kompressionsbüstenhalter (1), **dadurch gekennzeichnet, dass** der Kompressionsbüstenhalter (1) ein im Tragezustand um den Thorax der Trägerin angeordnetes durchgehendes Kompressionsband (3) umfasst, wobei das Kompressionsband (3) eine Aussparung (4), die einen Freiraum für eine Brust bildet, aufweist.

2. Kompressionsbüstenhalter nach Anspruch 1, **dadurch gekennzeichnet, dass** das Kompressionsband (3) einstückig ausgebildet ist.

3. Kompressionsbüstenhalter nach Anspruch 1 oder 2, **dadurch gekennzeichnet, dass** der Kompressionsbüstenhalter (1) an seinen beiden Enden jeweils ein Verschlussteil (7, 8) aufweist, wobei die Verschlussteile (7, 8) einander zugeordnet und miteinander verbindbar sind.

4. Kompressionsbüstenhalter nach einem der vorhergehenden Ansprüche, **dadurch gekennzeichnet, dass** der Kompressionsbüstenhalter (1) ein im Tragezustand um den Thorax der Trägerin angeordnetes durchgehendes Unterbrustband (5) umfasst.

5. Kompressionsbüstenhalter nach Anspruch 4, **dadurch gekennzeichnet, dass** das Unterbrustband (5) entlang seiner Oberkante mit der Unterkante des Kompressionsbands (3) durch eine Naht (11) verbunden ist.

6. Kompressionsbüstenhalter nach Anspruch 4 oder 5, **dadurch gekennzeichnet, dass** das Unterbrustband (5) in seiner den Thorax umgebenden Längsausdehnung und/oder seiner Querausdehnung zumindest zum Teil elastisch ist.

7. Kompressionsbüstenhalter nach einem der vorhergehenden Ansprüche, **dadurch gekennzeichnet, dass** das Kompressionsband (3) unterhalb der Aussparung (4) in seiner quer zur den Thorax umgebenden Längsausdehnung eine Breite (14) von mindestens 0,5 cm aufweist.

8. Kompressionsbüstenhalter nach Anspruch 3 bis 7, **dadurch gekennzeichnet, dass** der eine Verschlussteil (7) aus einem Hakenband und der andere Verschlussteil (8) einem Ösenband besteht.

9. Kompressionsbüstenhalter nach einem der vorhergehenden Ansprüche, **dadurch gekennzeichnet, dass** die Aussparung (4) an der dem Thorax abgewandten Seite mit einer Stofflage abgedeckt ist.

10. Kompressionsbüstenhalter nach Anspruch 9, **dadurch gekennzeichnet, dass** durch die Aussparung (4) im Kompressionsband (3) und der Stofflage einen Aufnahmeraum (13) für eine Brust gebildet wird.
